# EUROPÄISCHE PATENTANMELDUNG

(11) **EP 2 420 577 A1**
(43) Veröffentlichungstag der Anmeldung: **22.02.2012**
(21) Anmeldenummer: 10173593.4
(22) Anmeldetag: 20.08.2010
(51) Int. Cl.: C12Q 1/58

(54) **Verfahren zur Bestimmung von Harnstoff**

(71) Anmelder: Cytonet GmbH & Co. KG, 69469 Weinheim/Bergstrasse (DE)
(72) Erfinder: Aygen, Sitke, Dr., 51105 Köln (DE)
(74) Vertreter: Schrell, Andreas

(57) **Zusammenfassung**

Verfahren zur Bestimmung von Harnstoff in Plasmaproben umfassend folgende Schritte
a) Bereitstellen einer Plasmaprobe enthaltend Harnstoff
b) Zugabe einer Säure
c) Gefriertrocknen der Probe, um CO₂ zu entfernen und eine getrocknete Probe zu erhalten
d) Wiederauflösen der getrockneten Probe und Neutralisieren auf einen pH-Wert von 5 bis 7 mit einer Pufferlösung
e) Spülen mit Schutzgas
f) Zugabe von Urease, um CO₂ zu bilden
g) Inkubation
h) Zugabe einer Säure, um das gebildete CO₂ freizusetzen
i) Bestimmung des Isotopenverhältnisses des freigesetzten CO₂.

## Beschreibung

Die vorliegende Erfindung betrifft ein Verfahren zur Bestimmung von Harnstoff in Blutproben.

Harnstoff ist eine organische Verbindung, die im menschlichen Organismus ein Endprodukt des Stoffwechsels von Stickstoffverbindungen darstellt. Beim Menschen wird Harnstoff mit dem Urin ausgeschieden.

Die Harnstoffbildung erfolgt überwiegend in den Leberzellen und teilweise in den Nieren. Bei der Harnstoffbildung im Körper gibt es verschiedene Erkrankungen, teilweise erblich, die erhebliche gesundheitliche Schäden bewirken können. Die Bestimmung der Harnstoffbildung ist beispielsweise auch bei Lebertransplantationen oder der Transplantation von Leberzellen relevant.

Tuchman et al., Pediatric Research 2008 (64), Seite 213 beschreiben beispielsweise einen Defekt der N-Acetylglutamat-Synthase.

Zur Messung der Harnstoffbildung erhielten Patienten oral ¹³C markiertes Natriumacetat verabreicht, das im Körper zur Bildung von ¹³C markiertem Harnstoff führte; aus ¹³C markiertem Acetat entsteht ¹³CO₂, das in ¹³C-Carbamoyl-Phosphat und anschließend in ¹³C Harnstoff überführt wird.

Zur Bestimmung des ¹³C markierten Harnstoffes verwenden Tuchman et al. ein Verfahren mit folgenden Schritten:
Eine Plasmaprobe von 0,5 ml wurde mit 0,5 ml H₂O und 40 µl Perchlorsäure 60 % versetzt und präzipitiertes Protein abgetrennt. Anschließend ruhte der Behälter 30 Minuten um eine Freisetzung von CO₂ zu erlauben. Nach Überführen in ein neues Gefäß und Einstellen des pH in den Bereich 6 bis 7 mit 300 µl KOH 1 M wurde präzipitiertes Kaliumperchlorat abgetrennt. Mit Hilfe einer Ionenaustauschersäule wurde restliches Bicarbonat entfernt.

Die Säule wurde mit 1 ml HCl 10 mM gewaschen und das Eluat in einem Glasbehälter bei 80°C getrocknet. Die Probe ruhte über Nacht in einem verschlossenen Behälter, in dem ein Stück Gaze, das mit Natriumhydroxid getränkt war, mit eingeschlossen war, um Reste von CO₂ abzufangen.

Anschließend wurde der Behälter mit Helium gespült und das Gefäß mit einem Gummistopfen luftdicht verschlossen. Es wurden 400 µl Kaliumphosphatpuffer 0.5 M pH 6.0 mit 3 mg Urease-Enzym/400 µl durch den Gummistopfen injiziert. Nach einer Stunde erfolgte eine Zugabe von 100 µL Phosphorsäure 20 %, um CO₂ freizusetzten und die Urease-Reaktion zu stoppen. Das freigesetzte ¹³CO₂ wurde mit Hilfe eines isotope ratio mass spectrometer (IRMS) vermessen.

Die Untersuchungen der Anmelderin haben ergeben, dass das oben beschriebene Verfahren sehr empfindlich ist. Mehrere Quellen von Fremd-CO₂ können bei diesem Verfahren die gemessenen delta-Werte des aus Harnstoff entstandenen CO₂ verfälschen. Es ist darüber hinaus auch sehr aufwändig und langwierig. Wegen der niedrigen Ausbeute an Harnstoff ist ein größeres Volumen (0,5 ml) von Plasma notwendig. Dies kann bei Kindern Probleme hervorrufen. Auch bei einer Crossvalidierung mit USA und Europa haben sich nicht nachvollziehbare Differenzen in den Ergebnissen gezeigt.

Aufgabe der Erfindung war es, ein Verfahren bereitzustellen, das zumindest einige der Nachteile des bekannten Verfahrens überwindet.

Gelöst wird die Aufgabe durch ein Verfahren zur Bestimmung von Harnstoff in Plasmaproben, umfassend folgende Schritte:
a) Bereitstellen einer Plasmaprobe enthaltend Harnstoff
b) Zugabe einer Säure
c) Gefriertrocknen der Probe, um CO₂ zu entfernen und eine getrocknete Probe zu erhalten
d) Wiederauflösen der getrockneten Probe und Neutralisieren auf einen pH-Wert von 5 bis 7 mit einer Pufferlösung
e) Spülen mit Schutzgas
f) Zugabe von Urease, um CO₂ zu bilden
g) Inkubation
h) Zugabe einer Säure, um das gebildete CO₂ freizusetzen
i) Bestimmung des Isotopenverhältnisses des freigesetzten CO₂.

Ausgangspunkt für die Bestimmung des Harnstoffs in einer Plasmaprobe ist eine Plasmaprobe, die Harnstoff enthält. Plasmaproben können in bekannter Weise aus Blutproben gewonnen werden. Auf Grund der hohen Reproduzierbarkeit des erfindungsgemäßen Verfahrens genügen Plasmaproben mit einem Volumen im Bereich von 0,2 bis 0,3 ml, es können aber auch größere Mengen eingesetzt werden.

Die Plasmaprobe enthält zumindest Harnstoff mit einem natürlichen Isotopenverhältnis. Sie kann zusätzlich mit ¹³C angereichertem Harnstoff versetzt sein. Sie kann aber auch durch Verabreichung von ¹³C markierten Vorstufen beispielsweise Acetat oder Bicarbonat angereichert sein. Hiervon können verträgliche Salze, z.B. Na oder K-Salze verabreicht werden. Erfindungsgemäß wird der Harnstoff bestimmt durch Umsetzung des Harnstoffs mit Urease zur Freisetzung von CO₂, daher muss zunächst vorhandenes CO₂ entfernt werden.

Erfindungsgemäß wird hierzu zunächst eine Säure zugesetzt. Geeignet ist beispielsweise Phosphorsäure in einer Konzentration von etwa 20 %. Bezogen auf eine Plasmaprobe von 0,3 ml genügt die Säure in einer Menge von etwa 50 µl. Selbstverständlich können auch andere Säuren eingesetzt werden.

In bevorzugter Ausführungsform der Erfindung wird anschließend eine Filtration durchgeführt. Hierzu wird zur Fällung Lösungsmittel, beispielsweise Acetonitril und/oder Ameisensäure zugesetzt. Durch diese Filtration können Proteine und Lipide vom Plasma abgetrennt werden. Besonders geeignet hierfür sind sogenannte HybridSPE^{™} Säulen, die von der Firma SUPELCO erhältlich sind. Diese eignen sich insbesondere auch zu Entfernung von Phospholipiden.

Es hat sich jedoch gezeigt, dass auf den Schritt der Abtrennung der Proteine und Lipide aus dem Plasma auch verzichtet werden kann und trotzdem sehr gut reproduzierbare Werte erhalten werden. Der Verzicht auf den Filtrationsschritt spart zum einen Zeit, zum anderen aber auch Kosten, die für entsprechende Filter anfallen.

Ein wesentlicher Schritt des erfindungsgemäßen Verfahrens ist die anschließende Gefriertrocknung der Probe. Gefriertrocknen ist ein Verfahren, bei dem eine Probe eingefroren wird und im Vakuum das enthaltende Wasser sublimiert. Erfindungsgemäß eignet sich dieses Verfahren hervorragend zur Entfernung von Restmengen CO₂ in der Probe.

Die erhaltene Probe wird anschließend wieder aufgelöst und auf einen pH-Wert im Bereich von 5 bis 7, vorzugsweise 5 bis 6 eingestellt. Zur Einstellung eignen sich insbesondere Pufferlösungen, beispielsweise Phosphatpufferlösungen mit einem pH von 9,0. Auch andere Pufferlösungen können eingesetzt werden. Die in Tuchman et al. eingesetzte Kalilauge ist besonders ungünstig, da sie teilweise größere Mengen CO₂ enthält.

Die wieder aufgelöste Probe wird mit einem Schutzgas gespült, beispielsweise Helium, Stickstoff oder Argon und gasdicht verschlossen. Anschließend erfolgt eine Zugabe von Urease, um aus dem vorhandenen Harnstoff CO₂ zu bilden. Geeignet ist beispielsweise eine Urease, wie sie von der Firma Sigma erhältlich ist. Eine Menge von 20 bis 100 Einheiten Urease für eine Plasmaprobe von 0,3 ml hat sich als besonders geeignet erwiesen.

Anschließend erfolgt eine Inkubation der Lösung. Die Inkubation bei einer Temperatur von etwa 36°C für einen Zeitraum von etwa 60 Minuten hat sich als geeignet erwiesen.

Danach wird eine Säure zugesetzt, um die weitere Ureasereaktion zu stoppen. Zusätzlich wird durch die Zugabe der Säure das gebildete CO₂ freigesetzt. Im Hinblick auf die 0,3 ml große Plasmaprobe hat sich die Verwendung von 100 µl Phosphorsäure 20 %ig als geeignet erwiesen. In dem gasdichten Behälter ist nun CO₂ freigesetzt worden, das aus der Umsetzung des Harnstoffs im Plasma stammt. Die Menge an CO₂ kann über das Isotopenverhältnis des CO₂ bestimmt werden. Für diese Bestimmung eignet sich insbesondere IRMS. Bei der IRMS wird das Verhältnis zwischen ¹³C und ¹²C relativ zu einem Standard gemessen.

In einer Ausführungsform der Erfindung wird nach dem Wiederauflösen der getrockneten Proben diese erwärmt, beispielsweise in einem Ultraschallbad, um Reste von Fremd-CO₂ auszutreiben.

Zur Prüfung der Durchführung kann es sinnvoll sein, vor der Zugabe von Urease zu prüfen, ob die Lösung frei von CO₂ ist. Dies kann beispielsweise mittels IR-MS Spektroskopie erfolgen.

In vielen Ausführungsformen werden zur Messung der Kinetik der Harnstoffbildung ¹³C markierte Substanzen dem Patienten verabreicht, um die Bildung von ¹³C markiertem Harnstoff zu bestimmen. In einer bevorzugten Ausführungsform der Erfindung wird eine Kinetik dadurch bestimmt, dass zunächst eine Blutprobe entnommen wird, anschließend eine ¹³C markierte Verbindung dem Patienten verabreicht wird und dann die Bildung von ¹³C markiertem Harnstoff über einen Zeitraum durch mehrere Messungen nach Blutentnahmen überprüft wird.

Erfindungsgemäß können Plasmamengen von etwa 100 bis 200 oder 200 bis 300 ml eingesetzt werden, d.h. im Vergleich zum Verfahren von Tuchman, genügt eine Blutprobe mit halber Größe. Insbesondere wenn eine Kinetik bestimmt wird und das Verfahren bei Kindern angewandt wird, ist es vorteilhaft, wenn die Mengen an genommenem Blut besonders klein sind. Gegenüber dem Verfahren von Tuchman et al. hat das erfindungsgemäße Verfahren eine bessere Reproduzierbarkeit und ist weniger aufwändig.

Das Verfahren wird durch die folgenden Beispiele näher erläutert.

### Beispiel 1: Harnstoffisolation mit Filtration

Aus einer Blutprobe wurde Plasma gewonnen. 300 µl Plasma wurden mit 200 µl deionisiertem Wasser verdünnt. Zu der Probe wurden 50 µl Phosphorsäure 1 M sowie 100 µl Acetonitril mit 1 % Ameisensäure zugesetzt. In dem Gefäß bildete sich ein Niederschlag. Die Proben wurden über eine HybridSPE-Säule abfiltriert.

Das Filtrat wurde eingefroren und lyophilisiert. Die Probe wurde mit einer entgasten Phosphatpufferlösung 0,5 M pH 9 auf einen pH-Wert von 5,5 eingestellt. Der Probenbehälter (Vacutainer) wurde mit Heliumgas gespült. Anschließend erfolgte die Zugabe von 70 µl einer Lösung enthaltend 15 mg/ml Jack Bean Urease Typ III der Firma Sigma in Phosphatpuffer. Die Probe wurde für eine Stunde bei 36°C inkubiert. Anschließend wurden durch das Septum 60 µl Phosphorsäure 20 %ig injiziert, um die Ureasereaktion zu stoppen und CO₂ freizusetzen. Aus dem freigesetzten CO₂ erfolgte die Bestimmung des Isotopenverhältnisses mittels IRMS.

### Beispiel 2: Harnstoffisolation ohne Filtration

Das Verfahren wurde wie in Beispiel 1 durchgeführt, allerdings wurde auf die Zugabe von Acetonitril und Ameisensäure sowie die Filtration verzichtet, d.h. die Plasmaprobe wurde nach Zugabe von Säure direkt lyophilisiert. Das weitere Verfahren wurde identisch durchgeführt.

### Beispiel 3: Reproduzierbarkeit des Verfahrens

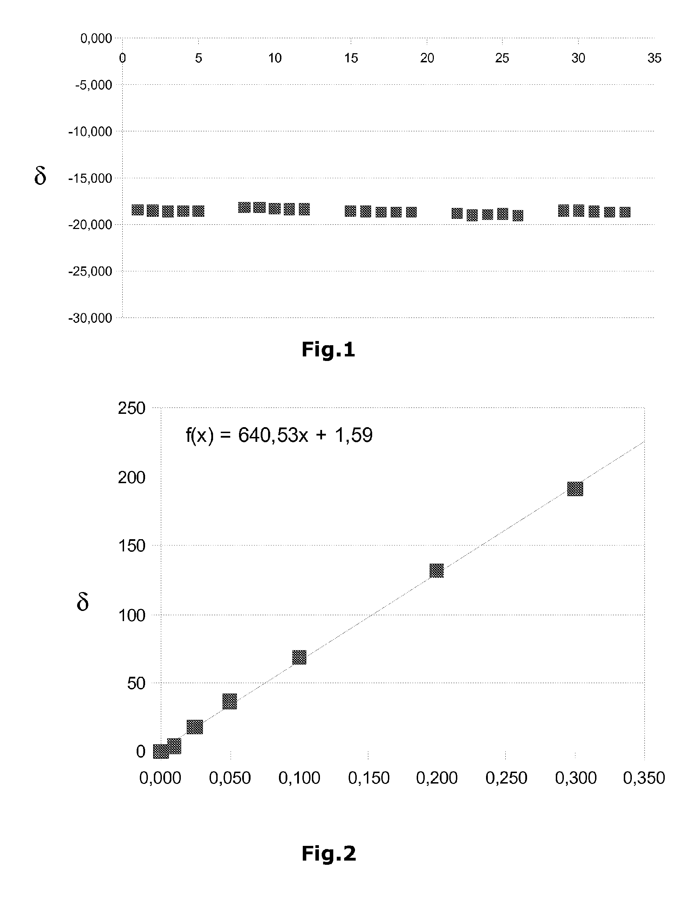

Eine Plasmaprobe wurde in fünf Proben unterteilt, die jeweils getrennt voneinander dem Verfahren gemäß Beispiel 1 unterzogen wurden. Jede erhaltene CO₂ Probe wurde fünfmal gemessen. Die Unterschiede sind minimal, vgl. Figur 1.

### Beispiel 4: Spiking-Experiment

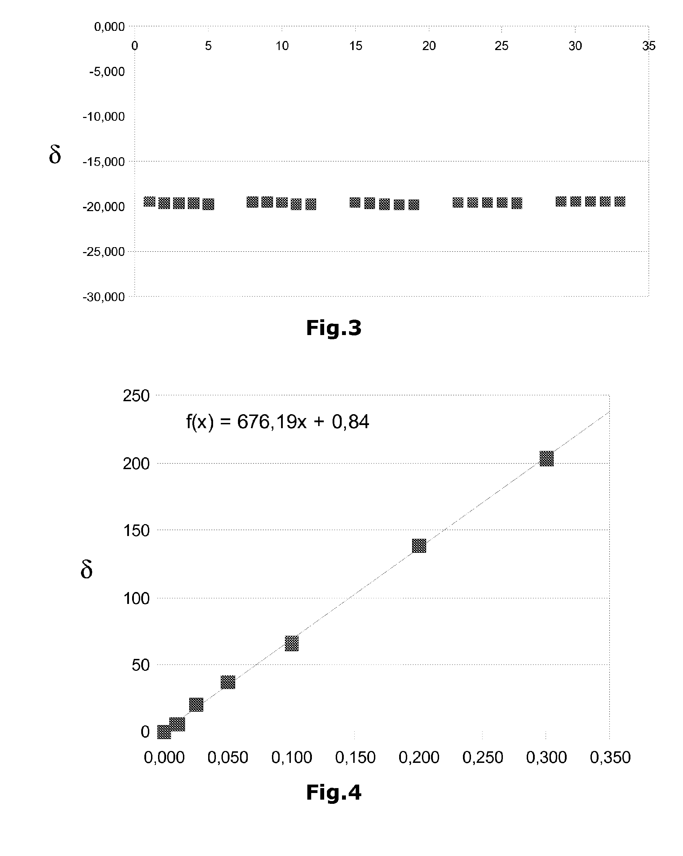

Den Plasmaproben aus Beispiel 1 wurden 99 %ig markierter ¹³C Harnstoff in Mengen von 0,01 mg, 0,25 mg, 0,5 mg, 0,1 mg, 0,2 mg, 0,3 mg zugesetzt und die Probe gemäß Verfahren 1 behandelt. Figur 3 zeigt die entsprechenden Messwerte. Die Eichkurve liegt auf einer Geraden mit einem Korrelationskoeffizienten von 0,99923.

### Beispiel 5: Reproduzierbarkeit

Die Messung der Reproduzierbarkeit gemäß Beispiel 3 wurde für das Verfahren ohne Filter gemäß Beispiel 2 wiederholt. Figur 3 zeigt die Ergebnisse. Auch hier besteht eine hervorragende Reproduzierbarkeit.

### Beispiel 6:

Das Spiking-Experiment gemäß Beispiel 4 wurde wiederholt, wobei das Verfahren gemäß Beispiel 2 eingesetzt wurde. Die entsprechende Eichgerade ist in Figur 4 ersichtlich. Ihr Korrelationskoeffizient lag bei R=0,99959.

## Patentansprüche

1. Verfahren zur Bestimmung von Harnstoff in Plasmaproben umfassend folgende Schritte
a) Bereitstellen einer Plasmaprobe enthaltend Harnstoff
b) Zugabe einer Säure
c) Gefriertrocknen der Probe, um CO₂ zu entfernen und eine getrocknete Probe zu erhalten
d) Wiederauflösen der getrockneten Probe und Neutralisieren auf einen pH-Wert von 5 bis 7 mit einer Pufferlösung
e) Spülen mit Schutzgas
f) Zugabe von Urease, um CO₂ zu bilden
g) Inkubation
h) Zugabe einer Säure, um das gebildete CO₂ freizusetzen
i) Bestimmung des Isotopenverhältnisses des freigesetzten CO₂.

2. Verfahren nach Anspruch 1, **dadurch gekennzeichnet, dass** vor dem Gefriertrocknen eine Filtration durchgeführt wird.

3. Verfahren nach Anspruch 1 oder 2, **dadurch gekennzeichnet, dass** nach Schritt d) die Probe eine Temperatur zwischen 40 und 70°C erwärmt wird.

4. Verfahren nach mindestens einem der Ansprüche 1 bis 3, **dadurch gekennzeichnet, dass** zur Bestimmung des Isotopenverhältnisses des CO₂ IRMS eingesetzt wird.

5. Verfahren nach mindestens einem der Ansprüche 1 bis 4, **dadurch gekennzeichnet, dass** vor der Entnahme einer Plasmaprobe ¹³C -markiertes Acetat verabreicht wird.

6. Verfahren nach mindestens einem der Ansprüche 1 bis 5, **dadurch gekennzeichnet, dass** die Probe in Schritt d) auf einen pH-Wert von 5 bis 6 eingestellt wird.

7. Verfahren zur Diagnostik des Harnstoffstoffwechsels umfassend die Schritte
- Entnehmen einer Blutprobe von einem Patienten,
- Gewinnung einer Plasmaprobe aus der Blutprobe,
- Bestimmung des Harnstoffs in der Plasmaprobe nach dem Verfahren gemäß einem der Ansprüche 1 bis 6,
- Verabreichen eines ¹³C markierten Harnstoffvorläufers,
- Entnahme von mindestens zwei Blutproben in einem zeitlichen Abstand
- Bestimmung des Harnstoffs nach Gewinnung von Plasmaproben aus den Blutproben durch ein Verfahren nach einem der Ansprüche 1 bis 6.

8. Verfahren nach Anspruch 7, **dadurch gekennzeichnet, dass** nach dem Verabreichen der Harnstoffvorläufer, die mindestens zwei Blutproben über einen Zeitraum von mindestens 120 Minuten, bevorzugt mindestens 240 Minuten entnommen werden.

9. Verfahren nach Anspruch 7 oder 8, **dadurch gekennzeichnet, dass** zwischen zwei Entnahmen von Blutproben ein Zeitraum von 10 bis 20 Minuten liegt.
